# EUROPEAN PATENT APPLICATION

(11) **EP 3 689 845 A1**
(43) Date of publication of application: **05.08.2020**
(21) Application number: 17926908.9
(22) Date of filing: 29.09.2017
(51) Int. Cl.: C07C 29/149, C07C 29/151, C07C 31/08

(54) **METHOD FOR DIRECTLY PRODUCING ETHANOL FROM SYNGAS**

(71) Applicant: Dalian Institute Of Chemical Physics, Chinese Academy of Sciences, Liaoning 116023 (CN)
(72) Inventor: LIU, Hongchao, Dalian Liaoning 116023 (CN); ZHU, Wenliang, Dalian Liaoning 116023 (CN); LIU, Zhongmin, Dalian Liaoning 116023 (CN); LIU, Yong, Dalian Liaoning 116023 (CN); LIU, Shiping, Dalian Liaoning 116023 (CN); WEN, Fuli, Dalian Liaoning 116023 (CN); NI, Youming, Dalian Liaoning 116023 (CN); MA, Xiangang, Dalian Liaoning 116023 (CN)
(74) Representative: Witte, Weller & Partner Patentanwälte mbB
(86) International application number: PCT/CN2017/104554
(87) International publication number: WO 2019/061342

(57) **Abstract**

Disclosed herein is a method for directly producing ethanol from syngas, whose the reaction process is carried out in three reaction zones, and the method comprises: feeding a raw material containing syngas and dimethyl ether into a first reaction zone to contact with a solid acid catalyst, reacting; allowing the effluent from the first reaction zone to enter a second reaction zone to contact with a metal catalyst and react; separating the effluent from the second reaction zone to obtain product ethanol and by-product methanol; allowing by-product methanol to enter a third reaction zone to perform a dehydration reaction to obtain dimethyl ether, and allowing the obtained dimethyl ether to enter the first reaction zone to recycle the reaction. The present invention provides a novel method for directly converting syngas to ethanol. According to the method of the present invention, an ethanol product can be directly produced by using syngas as a raw material. Meanwhile, the method of the present invention has a simple processing procedure, low energy consumption, low production costs, and high product selectivity, and has great industrial application prospects.

## Description

### TECHNICAL FIELD

The present invention relates to a method for converting syngas into ethanol.

### BACKGROUND

Ethanol is recognized as an environmentally friendly clean fuel in the world. It can be used directly as a liquid fuel or mixed with gasoline to reduce the emissions of carbon monoxide, hydrocarbons, particulate matter, nitrogen oxides and benzene-based harmful substances in automobile exhaust, which can effectively improve China's Environmental quality is of great significance for solving China's air pollution problems and achieving sustainable development. Existing ethanol production processes mainly include a sugar or cellulose fermentation method based on a biomass route and an ethylene hydration method based on a petroleum route. In recent years, China's fuel ethanol production and sales have grown rapidly, and it has become the world's third largest fuel ethanol producer after the United States and Brazil. However, biosynthetic fuel ethanol is limited by the characteristics of the shortage of raw materials and low energy density, which makes it difficult to develop on a large scale. Based on China's energy structure of "poor oil, little gas, and relatively abundant coal resources" and the current status of increasing dependence on foreign petroleum, there is an urgent need to develop novel processes for synthesizing ethanol from coal or biomass-based syngas to reduce China's dependence on petroleum to promote the diversification of China's energy.

The production of a large variety of basic chemical raw materials and high value-added fine chemicals using syngas as raw materials has been a hot topic in the field of catalysis. The direct preparation of ethanol from syngas is a novel process for ethanol preparation in recent years. From the point of view of process and cost, the process of direct preparation of ethanol from syngas is short, the operating cost is relatively economical, and the investment cost is low. However, from the perspective of thermodynamics and kinetics, it is difficult for the reaction to stay on the target product, i.e. ethanol. Because the direct preparation of ethanol from syngas is a strong exothermic reaction, the first problem is to choose a catalyst with good catalytic performance, high selectivity and strong resistance. From the actual reaction results, the products are widely distributed. Not only there are a large number of C2 oxygenated byproducts such as acetaldehyde and acetic acid, but also C2-C5 alkanes and olefins. The selectivity of ethanol is not ideal and the yield is low.

The rhodium-based catalysts has attracted widespread attention from researchers at home and abroad because of its performance in the selective synthesis of C2 oxygenated compounds, which is one of the relatively important research directions for C1 chemistry in recent years. However, the use of precious metal such as rhodium has greatly increased the production cost of ethanol, and the production of rhodium is limited. There is great difficulty in large-scale promotion and application, which has become the bottleneck of the industrialization of this process route. Significantly reducing the use of rhodium or replacing rhodium with non-precious metal catalysts is an effective way to promote the industrialization of this technology, but progress is currently relatively slow.

CN103012062A discloses a process for the indirect production of ethanol from syngas, which comprises synthesizing methanol from a syngas raw material formed by mixing hydrogen and carbon monoxide, dehydrating methanol to prepare dimethyl ether, and then dimethyl ether being mixed with carbon monoxide and hydrogen to perform carbonylation reaction to obtain methyl acetate, purifying methyl acetate and then being hydrogenated, and purifying the hydrogenated product to obtain an ethanol product. The entire process includes process units such as synthesis and separation of methanol, synthesis and separation of dimethyl ether, carbonylation and separation of dimethyl ether, and hydrogenation and separation of methyl acetate. The present invention provides a method for directly producing ethanol from syngas. The method uses syngas as a raw material and integrates methanol synthesis, the preparation of dimethyl ether from methanol, the preparation of methyl acetate through dimethyl ether carbonylation, and the preparation of ethanol through methyl acetate hydrogenation, so as to realize the direct production of ethanol from syngas. The present invention not only reduces the methanol synthesis unit and the corresponding separation unit, but also reduces the separation unit for the preparation of methyl acetate through dimethyl ether carbonylation, so that the present invention has mild reaction conditions, simple process, low equipment investment cost and reduced energy consumption, and has important application prospects.

### SUMMARY OF THE INVENTION

The purpose of the present invention is to overcome some or all of the problems in the prior art, and provide a novel technology for syngas conversion and a method for ethanol production, by which the directional conversion of syngas to ethanol can be achieved.

To this end, the present invention provides a method for directly producing ethanol from syngas, whose reaction process is carried out in three reaction zones and the method comprises:
a) feeding a raw material containing syngas and dimethyl ether into a first reaction zone to contact with a solid acid catalyst in the first reaction zone, reacting to obtain an effluent containing methyl acetate and/or acetic acid;
b) allowing the effluent from the first reaction zone to enter a second reaction zone to contact with a metal catalyst in the second reaction zone and react to obtain an effluent containing methanol and ethanol;
c) separating the effluent from the second reaction zone to obtain product ethanol and by-product methanol;
d) allowing methanol from step c) to enter a third reaction zone to perform a dehydration reaction to obtain dimethyl ether, and allowing the obtained dimethyl ether to enter the first reaction zone to recycle the reaction;
wherein the volume content of syngas in the raw material is in a range from 10% to 100%, the volume content of dimethyl ether is in a range from 0% to 90%, and the volume ratio of carbon monoxide to hydrogen in the syngas is in a range from 0.1 to 10;
the reaction temperature in the first reaction zone and the second reaction zone is in a range from 180°C to 300°C, and the reaction pressure is in a range from 0.5MPa to 20 MPa;
the reaction temperature in the third reaction zone is in a range from 180°C to 420°C, and the reaction pressure is in a range from 0.1MPa to 4 MPa.

Preferably, the solid acid catalyst in the first reaction zone comprises one or more molecular sieves selected from FER zeolite molecular sieve, MFI zeolite molecular sieve, MOR zeolite molecular sieve, ETL zeolite molecular sieve, MFS zeolite molecular sieve, MTF zeolite molecular sieve, EMT zeolite molecular sieve and molecular sieve products obtained by modifying the zeolite molecular sieves using pyridine or elements other than the framework constituent elements.

Preferably, the solid acid catalyst is a hydrogen-type product of the zeolite molecular sieve, or is composed of ranging from 10% to 95% by weight of the hydrogen-type product and a remaining matrix, or is a molecular sieve product obtained by modifying the hydrogen-type product with pyridine, wherein the matrix is one or more selected from alumina, silica, kaolin and magnesia.

Preferably, the metal catalyst in the second reaction zone is a copper-based catalyst.

Preferably, the first reaction zone and/or the second reaction zone are in a fixed bed reactor, and the fixed bed reactor is preferably a tubular fixed bed reactor.

Preferably, the first reaction zone and the second reaction zone are in the same fixed reactor, or the first reaction zone and the second reaction zone are respectively in different reactors connected in series.

Preferably, the syngas in the raw material is composed of ranging from 50 to 100% by volume of carbon monoxide and hydrogen and ranging from 0% to 50% by volume of one or more inactive gases selected from nitrogen, helium, argon and carbon dioxide.

Preferably, the catalyst in the third reaction zone is a solid acid catalyst for preparing dimethyl ether from methanol.

Preferably, the third reaction zone is in a fixed bed reactor, especially in a tubular fixed bed reactor.

Preferably, the reaction temperature in the first reaction zone is in a range from 190°C to 290°C and the reaction pressure is in a range from 1 MPa to 15 MPa; the reaction temperature in the second reaction zone is in a range from 190°C to 290°C and the reaction pressure is in a range from 1.0 MPa to 15.0 MPa; the reaction temperature in the third reaction zone is in a range from 200°C to 400°C, and the reaction pressure is in a range from 0.2 MPa to 3 MPa.

The present invention includes but is not limited to the following beneficial effects:
1. This is provided a method for directly producing ethanol from syngas. The method integrates the processes of synthesizing methanol, preparing dimethyl ether from methanol, preparing methyl acetate by carbonylation of dimethyl ether and preparing ethanol by hydrogenation of methyl acetate, reduces methanol synthesis, the separation unit for preparing methyl acetate by carbonylation of dimethyl ether. The equipment investment cost is reduced by ranging from 5% to 10%, and the energy consumption is reduced by ranging from 10% to 20%. At the same time, both the reaction of methyl acetate hydrogenation to produce ethanol and methanol is provided on the metal catalyst. And the process of hydrogenation of synthesis gas to methanol.
2. The method has the advantages of low equipment investment, mild reaction conditions, simple process and the like, and has important application prospects.

### BRIEF DESCRIPTION OF THE DRAWINGS

FIG. 1 is a flow chart of the preparation of ethanol from syngas according to an embodiment of the present invention, wherein the first reaction zone and the second reaction zone are in the same reactor.
FIG. 2 is a flow chart of the preparation of ethanol from syngas according to another embodiment of the present invention, wherein the first reaction zone and the second reaction zone are in different reactors.

### DETAILED DESCRIPTION OF THE EMBODIMENT

The method of the present invention includes the following processes: contacting syngas gaseous materials containing dimethyl ether with a solid acid catalyst in the first reaction zone to react to obtain an oxygen-containing compound of methyl acetate; and then contacting the syngas and methyl acetate with a metal catalyst in the second reaction zone to react to generate methanol and ethanol, subsequently separating ethanol as a product, dehydrating methanol in the third reaction zone to form dimethyl ether, recycling the resulting dimethyl ether into the reaction system and further coverting it as raw material together with syngas. The method can realize the high-efficiency conversion of a single syngas to produce ethanol, has high selectivity of ethanol, reduces related operation units, reduces equipment investment and energy consumption, and has a simple entire process and good application prospects.

More specifically, in the method for directly producing ethanol from the syngas according to the present invention, the reaction process is carried out in three reaction zones, and the method comprises:
a) feeding a raw materials syngas containing dimethyl ether into a first reaction zone to contact with a solid acid catalyst in the first reaction zone, reacting to obtain an effluent containing methyl acetate and/or acetic acid;
b) allowing the effluent from the first reaction zone to enter a second reaction zone to contact with a metal catalyst in the second reaction zone and react to obtain an effluent containing methanol and ethanol;
c) separating the effluent from the second reaction zone to obtain product ethanol and by-product methanol;
d) allowing methanol from step c) to enter a third reaction zone to perform a dehydration reaction to obtain dimethyl ether, and allowing the obtained dimethyl ether to enter the first reaction zone to recycle the reaction;
wherein the volume content of syngas in the raw material is in a range from 10% to 100%, the volume content of dimethyl ether is in a range from 0% to 90%, and the volume ratio of carbon monoxide to hydrogen in the syngas is in a range from 0.1 to 10;
the reaction temperature in the first reaction zone and the second reaction zone is in a range from 180°C to 300°C, and the reaction pressure is in a range from 0.5 MPa to 20 MPa;
the reaction temperature in the third reaction zone is in a range from 180°C to 420°C, and the reaction pressure is in a range from 0.1 MPa to 4 MPa.

In the method of the present invention, preferably, the solid acid catalyst in the first reaction zone comprises any one or any combination of zeolite molecular sieves of FER, MFI, MOR, ETL, MFS, MTF or EMT structures, or products obtained by modification of elements other than the constituent elements (such as Fe, Ga, Cu, Ag, etc.) of molecular sieve framework that meets the above characteristics or by modification of pyridine, or a mixture of multiple molecular sieves that meet the above characteristics.

Preferably, the solid acid catalyst is a hydrogen-type product of the zeolite molecular sieve, or is composed of ranging from 10% to 95% by weight of the hydrogen-type product and a remaining matrix, or is a molecular sieve product obtained by modification of hydrogen-type product with pyridine; more preferably, the matrix is any one or a mixture of any one of alumina, silica, kaolin and magnesia.

In the method of the present invention, preferably, the metal catalyst in the second reaction zone is a copper-based catalyst having methanol synthesis and hydrogenation performance.

Preferably, reactors for the first reaction zone and the second reaction zone each adopt a fixed bed reactor, preferably a tubular fixed bed tube reactor.

In the method of the present invention, the first reaction zone and the second reaction zone may be in the same reactor, or the first reaction zone and the second reaction zone may be in different reactors connected in series.

In the method of the present invention, in addition to carbon monoxide and hydrogen, the syngas raw material may also contain any one or more inactive gases selected from nitrogen, helium, argon and carbon dioxide. Preferably, the volume content of carbon monoxide and hydrogen is in a range from 50% to 100%; the volume percentage content of any one or more gases selected from nitrogen, helium, argon and carbon dioxide in the syngas raw material is in a range from 0% to 50%.

In the method of the present invention, preferably, the catalyst in the third reaction zone is a solid acid catalyst for preparing dimethyl ether from methanol, and preferably, the reactor may be a conventional fixed bed reactor, or a tubular fixed bed reactor.

In a further preferred embodiment, the reaction conditions in the first reaction zone are as follows: the reaction temperature is in a range from 190°C to 290°C, the reaction pressure is in a range from 1.0 MPa to 15.0 MPa; the reaction conditions in the second reaction zone are as follows: the reaction temperature is in a range from 190°C to 290°C, the reaction pressure is in a range from 1.0 MPa to 20.0 MPa; the reaction conditions in the third reaction zone are as follows: the reaction temperature are in a range from 200°C to 400°C, the reaction pressure is in a range from 0.2 MPa to 3 MPa.

The present invention is specifically illustrated by the following examples, but the present invention is not limited to these examples.

### Raw Material Source of Molecular Sieve

In the course of the experiment, part of the molecular sieve materials can be directly purchased commercially; part of the molecular sieve materials can be synthesized according to existing related literatures, and the specific sources were shown in Table 1.

**Table 1: Sources of different molecular sieve materials and ratio of silicon-aluminum**

| **Molecular sieve material** | **Way of obtaining** | **Source** | **Si/Al ratio** |
|---|---|---|---|
| NaMOR (mordenite) | purchase | The Catalyst Plant of Nankai University | 6.5 |
| NaMOR (mordenite) | purchase | The Catalyst Plant of Nankai University | 15 |
| NaSM-35 | purchase | The Catalyst Plant of Aoke | 79 |
| NaZSM-5 | purchase | The Catalyst Plant of Nankai University | 50 |
| NaEMT | synthesis | Dalian Institute of Chemical Physics. | 4 |
| NaEMT | synthesis | Dalian Institute of Chemical Physics. | 25 |
| Na-EU-12 | synthesis | Dalian Institute of Chemical Physics. | 10 |
| Na-MCM-65 | synthesis | Dalian Institute of Chemical Physics. | 50 |
| Na-MCM-35 | synthesis | Dalian Institute of Chemical Physics. | 100 |
| Na-M-MOR* | synthesis | Dalian Institute of Chemical | 16.5 |
| | | Physics. | |

| | | | |
|---|---|---|---|
| * Na-M-MOR represents mordenite modified by elements other than the framework constituent elements prepared by in-situ synthesis, wherein M represents a modified metal atom, and the molecular sieve modified by Fe, Ga, Cu, and Ag metals were prepared during the preparation process respectively, wherein the content of the modified metal was 0.9%. | | | |

### Solid Acid Catalyst

The hydrogen-type sample was prepared as follows:
The Na-type molecular sieve in Table 1 were subjected to NH₄NO₃ ion exchange, dried and roasted to obtain hydrogen-type molecular sieve. For example, a preparation process for a typical hydrogen-type sample was as follows: in a hydrothermal synthesis kettle, NaMOR molecular sieve powder was added to a pre-configured 1mol/L NH₄NO₃ aqueous solution with a solid-liquid mass ratio of 1:10, and was exchanged for 2 hours at 80°C in the stirring state, filtered under vacuum and washed with water. After three consecutive exchange reactions, it was dried at 120°C overnight and calcined for 4 hours at 550°C to obtain the required catalyst sample HMOR.

Matrix-containing shaped hydrogen-type samples were prepared by extrusion molding. For example, the preparation process for a typical shaped sample was as follows: 80 g of Na-MOR and 20 g of alumina were thoroughly mixed, and 5-15% nitric acid was added for kneading. The sample that was kneaded into a ball was extruded and shaped by an extruder. The extruded sample was dried at 120°C, and calcined at 550°C for 4 hours, and then a hydrogen-type sample preparation method was used to prepare matrix-containing shaped hydrogen-type samples.

Pyridine-modified hydrogen-type samples were prepared. The typical preparation process was as follows: 10g of hydrogen-type sample was charged into a reaction tube, and gradually heated to 300-550°C under a nitrogen atmosphere of 100mL/min, maintained for 2-6 hours, and then carried pyridine by nitrogen and treated at 200-400°C for 2-8 hours, to obtain pyridine modified samples. The samples were labeled with H-M-py, where M represents the name of the molecular sieve.

The series of samples prepared according to the above methods were shown in Table 2.

**Table 2: The number of the prepared sample and composition of the sample**

| Catalyst No. | Catalyst | Si/Al ratio of molecular sieve | Molecular sieve content | Matrix type | Matrix content |
|---|---|---|---|---|---|
| 1# | H-MOR | 6.5 | 100% | - | 0% |
| 2# | H-MOR | 6.5 | 50% | silica + alumina + magnesia (mass ratio 2: 2: 1) | 50% |
| 3# | H-MOR | 15 | 80% | alumina | 20% |
| 4# | H-ZSM-35 | 79 | 80% | kaolin | 20% |
| 5# | H-ZSM-5 | 50 | 70% | alumina | 30% |
| 6# | H-EMT | 4 | 80% | alumina | 20% |
| 7# | H-EMT | 25 | 80% | alumina | 20% |
| 8# | H-EU-12 | 10 | 80% | alumina | 20% |
| 9# | H-MCM-65 | 50 | 80% | alumina | 20% |
| 10# | H-MCM-35 | 100 | 90% | alumina | 10% |
| 11# | H-MOR-py | 15 | 80% | alumina | 20% |
| 12# | H-EMT-py | 25 | 80% | alumina | 20% |
| 13# | H-Fe-MOR | 16.5 | 100 | - | 0% |
| 14# | H-Cu-MOR | 16.5 | 100 | - | 0% |
| 15# | H-Ag-MOR | 16.5 | 100 | - | 0% |
| 16# | H-Ga-MOR | 16.5 | 100 | - | 0% |

### Metal Catalyst

The metal catalyst was a copper-based catalyst, which was prepared as follows: in a beaker, 96.80 g of Cu(NO₃)₂·3H₂O, 15.60 g of Zn (NO₃)₂·6H₂O, and 14.71 g of Al(NO₃)₃·9H₂O were dissolved in 2000 ml of deionized water, a mixed metal nitrate aqueous solution was obtained. In another beaker, 72.62 g of strong ammonia water (25-28%) was diluted with 1500 ml of deionized water, and was vigorously stirred the ammonia solution at room temperature, and then the obtained mixed metal nitrate aqueous solution was slowly added to the ammonia solution, the addition time was about 60min. It was filtered to obtain a precipitate, and the pH value of the precipitate was adjusted to 10.0 with another aqueous ammonia solution. After stirring for 200 minutes, it was left to age for 36 hours. Then, the precipitate was washed with deionized water to neutrality, and centrifuged. The obtained precipitate was dried in an oven at 120°C for 24 hours. The dried sample was placed in a muffle furnace, heated to 400°C at a temperature increase rate of 1°C/min, and roasted for 5 hours to obtain a roasted sample. 1.41 g of Mn(NO₃)₂·4H₂O and 1.36 g of Ni(NO₃)₂·4H₂O were then dissolved in 50 ml of deionized water, and the aqueous solution of manganese and nickel was loaded on the roasted sample by the dipping method, and evaporated at 80°C to remove excess solvent. It was dried in an oven at 120°C for 24 hours. After drying, the sample was placed in a muffle furnace, heated to 400°C at a heating rate of 1°C/min, and calcined for 3 hours to obtain a catalyst sample, which was recorded as catalyst B.

### Catalyst for Preparing Dimethyl ether From Methanol

D803C-III01 (commercial catalyst, DICP) was used. The catalyst was a mixture of ZSM-5 molecular sieve and γ-alumina in a ratio of 50:50, and was recorded as catalyst C.

### EXAMPLE 1

Catalyst 11# was used in the first reaction zone, catalyst B (copper-based catalyst) was used in the second reaction zone, and catalyst C was used in the third reaction zone.

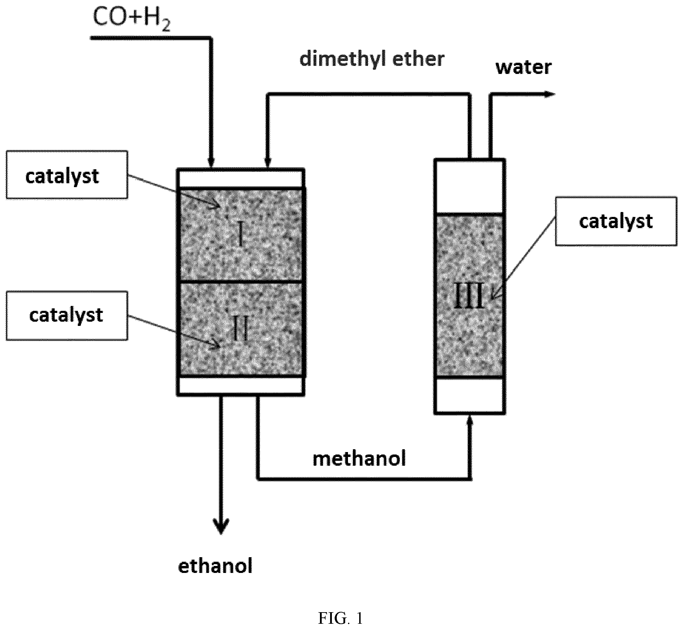

In the fixed-bed reactor, syngas containing CO and H₂ together with dimethyl ether (DME) passed through the first reaction zone and the second reaction zone. The first reaction zone and the second reaction zone were located in the same reactor, wherein the whole of part of dimethyl ether was produced from methanol generated from CO and H₂ in the second raction zone through the dehydration reaction in the third reaction zone. The specific reaction scheme was shown in FIG. 1, wherein the syngas and dimethyl ether as raw materials were allowed to enter the first reaction zone I to contact with the solid acid catalyst 11# in the first reaction zone and react to obtain an effluent containing methyl acetate and/or acetic acid; the effluent from the first reaction zone was allowed to enter the second reaction zone II to contact with the metal catalyst B in the second reaction zone and react to obtain an effluent containing methanol and ethanol; the effluent from the second reaction zone was separated to obtain product ethanol and by-product methanol; methanol from the previous step was allowed to enter the third reaction zone III to contact the catalyst C and perform a dehydration reaction to obtain dimethyl ether; and the obtained dimethyl ether was allowed to enter the first reaction zone to recycle the reaction.

The reaction conditions were as follows: catalyst 11 # and catalyst B were charged into the first reaction zone and the second reaction zone of the reactor from top to bottom, respectively, and 3g and 7g were charged respectively. Catalyst C in the third reaction zone was charged with 5g. The molar ratio of CO, DME and H₂ was 2: 1: 12. The feed of dimethyl ether was 3 g/h, the reaction temperature was 190°C, 215°C, 245°C, 275°C, and the reaction pressure was 5MPa. The reaction results were shown in Table 3.

**Table 3: Reaction results at different reaction temperatures**

| Reaction temperature /°C | Percent conversion of dimethyl ether (%) | Percent . conversion of carbon monoxide (%) | Selectivity for methanol (%) | Selectivity for ethanol(%) | Selectivity for methyl acetate(%) | Selectivity for ethyl acetate (%) | Selectivity for others (%) |
|---|---|---|---|---|---|---|---|
| 190 | 11.3 | 6.05 | 37.81 | 50.41 | 4.05 | 7.72 | 0.01 |
| 215 | 28.5 | 16.3 | 44.31 | 55.65 | 0.0 | 0.0 | 0.04 |
| 245 | 60.3 | 38.3 | 46.90 | 52.58 | 0.0 | 0.0 | 0.52 |
| 275 | 92.3 | 73.2 | 51.40 | 47.54 | 0.0 | 0.0 | 1.06 |

The methanol generated in the second reaction zone and unreacted dimethyl ether were recycled into the first reaction zone as raw materials after reacting in the third reaction zone, and the reaction temperature in the third reaction zone was 300°C.

### EXAMPLE 2

Different catalysts (1-10# and 12-16#, see Table 4) were used in the first reaction zone, catalyst B was used in the second reaction zone, and catalyst C was used in the third reaction zone.

In the fixed-bed reactor, syngas containing CO and H₂ together with dimethyl ether (DME) passed through the first reaction zone and the second reaction zone. The first reaction zone and the second reaction zone were located in the same reactor (the specific reaction process was shown in FIG. 1 and Example 1), wherein dimethyl ether was produced from methanol generated from CO and H₂ in the second raction zone through the dehydration reaction in the third reaction zone. The reaction conditions were as follows: different catalysts (1-10# and 12-16#, see Table 4) and catalyst B were charged into the first reaction zone and the second reaction zone of the reactor from top to bottom, respectively, and 3g and 7g were charged respectively. The molar ratio of CO, DME and H₂ was 2: 1: 12. The feed of dimethyl ether was 3 g/h, the reaction temperature was 215°C, and the reaction pressure was 5MPa. The reaction results were shown in Table 4.

| Catalyst | Percent conversion of dimethyl ether (%) | Percent . conversion of carbon monoxide (%) | Selectivity for methanol (%) | Selectivity for ethanol(%) | Selectivity for methyl acetate(%) | Selectivity for ethyl acetate (%) | Selectivity for others (%) |
|---|---|---|---|---|---|---|---|
| 1# | 15.3 | 9.70 | 46.87 | 50.83 | 0.0 | 0.0 | 2.30 |
| 2# | 9.5 | 6.80 | 49.90 | 48.60 | 0.0 | 0.0 | 1.80 |
| 3# | 12.3 | 8.20 | 48.12 | 49.98 | 0.0 | 0.0 | 1.90 |
| 4# | 4.7 | 4.40 | 56.57 | 41.92 | 0.0 | 0.0 | 1.51 |
| 5# | 6.2 | 5.15 | 53.61 | 26.69 | 0.0 | 0.0 | 19.70 |
| 6# | 9.3 | 6.70 | 50.06 | 47.84 | 0.0 | 0.0 | 2.10 |
| 7# | 12.3 | 8.20 | 48.12 | 50.38 | 0.0 | 0.0 | 1.50 |
| 8# | 5.8 | 4.95 | 54.28 | 45.61 | 0.0 | 0.0 | 0.11 |
| 9# | 2.3 | 3.20 | 65.94 | 33.74 | 0.0 | 0.0 | 0.32 |
| 10# | 3.2 | 3.65 | 61.34 | 37.76 | 0.0 | 0.0 | 0.90 |
| 12# | 30.8 | 17.45 | 44.08 | 55.87 | 0.0 | 0.0 | 0.05 |
| 13# | 13.8 | 8.38 | 47.92 | 50.38 | 0.0 | 0.0 | 1.70 |
| 14# | 14.7 | 9.10 | 47.33 | 50.87 | 0.0 | 0.0 | 1.80 |
| 15# | 13.1 | 8.65 | 46.82 | 51.20 | 0.0 | 0.0 | 1.98 |
| 16# | 13.9 | 8.47 | 47.33 | 51.38 | 0.0 | 0.0 | 1.29 |

### EXAMPLE 3

Similar to the procedure of Example 1, in the fixed-bed reactor, the reaction temperature was 215°C, and the reaction pressures were 1, 8 and 15 MPa, respectively. The other reaction conditions were the same as in Example 1. The reaction results when the mixed gas containing CO and H₂ together with dimethyl ether passed through the first reaction zone and the second reaction zone were shown in Table 5.

**Table 5: Reaction results at different reaction pressures**

| Reaction pre s sure/MP a | Percent conversion of dimethyl ether (%) | Percent conversion of carbon monoxide (%) | Selectivity for methanol (%) | Selectivity for ethanol(%) | Selectivity for methyl acetate(%) | Selectivity for ethyl acetate (%) | Selectivity for others (%) |
|---|---|---|---|---|---|---|---|
| 1 | 17.8 | 11.25 | 47.89 | 38.97 | 4.18 | 8.94 | 0.02 |
| 8 | 37.5 | 22.26 | 45.23 | 54.77 | 0.00 | 0.00 | 0.00 |
| 15 | 45.0 | 25.82 | 44.39 | 56.61 | 0.00 | 0.00 | 0.00 |

### EXAMPLE 4

Similar to the procedure of Example 1, the first reaction zone and the second reaction zone were in the same reactor, the molar ratio of CO, DME and H₂ was 2: 1: 12. The feed of dimethyl ether was 3 g/h. The reaction temperature and the reaction pressure were 215°C and 5 MPa respectively. The first reaction zone was filled with catalyst 11 # and the second reaction zone was filled with catalyst B. The specific loading amount was shown in Table 6, and the reaction results were shown in Table 6.

**Table 6: Reaction results when the first reaction zone and the second reaction zone were filled with different proportions of catalyst**

| Catalyst 11# (g) | Catalyst B (g) | Percent conversion of dimethyl ether (%) | Percent conversion of carbon monoxide (%) | Selectivity for methanol (%) | Selectivity for ethanol (%) | Selectivity for methyl acetate (%) | Selectivity for ethyl acetate (%) | Selectivity for others (%) |
|---|---|---|---|---|---|---|---|---|
| 0 | 10 | 0 | 21.89 | 99.5 | 0 | 0 | 0 | 0.5 |
| 3 | 0 | 26.5 | 13.25 | 0.0 | 0.0 | 99.8 | 0 | 0.2 |
| 5 | 5 | 45.5 | 27.26 | 42.51 | 42.59 | 6.31 | 8.56 | 0.03 |
| 8 | 2 | 75.0 | 41.39 | 30.85 | 39.60 | 13.23 | 16.32 | 0.00 |
| 10 | 0 | 99.5 | 49.75 | 0.0 | 0.0 | 99.8 | 0.00 | 0.1 |

### EXAMPLE 5

Similar to the procedure of Example 1, the first reaction zone and the second reaction zone were in the same reactor. The reaction conditions were as follows: catalyst 11 # and catalyst B were filled with 3g and 7g respectively. The mixed gas containing CO, DME and H₂, DME entered the first reaction zone, the molar ratio of CO, DME and H₂ was 1: 1: 12, 4: 1: 12 and 10: 1: 12, respectively. The feed of dimethyl ether was 3 g/h. When the temperature of the reaction zone was maintained at 215°C and the reaction pressure was 5 MPa, the reaction results were shown in Table 7.

**Table 7: Reaction results when the ratio of CO and dimethyl ether as raw materials was different**

| CO: DME | Percent conversion of dimethyl ether (%) | Percent conversion of carbon monoxide (%) | Selectivity methanol (%) | Selectivity for ethanol (%) | Selectivity for methyl acetate (%) | Selectivity for ethyl acetate (%) | Selectivity for others (%) |
|---|---|---|---|---|---|---|---|
| 1:1 | 10.5 | 12.5 | 54.4 | 45.6 | 0.0 | 0.0 | 0.01 |
| 4: 1 | 42.8 | 11.5 | 51.9 | 48.1 | 0.0 | 0.0 | 0.04 |
| 10:1 | 57.8 | 6.11 | 51.4 | 48.6 | 0.0 | 0.0 | 0.07 |

### EXAMPLE 6

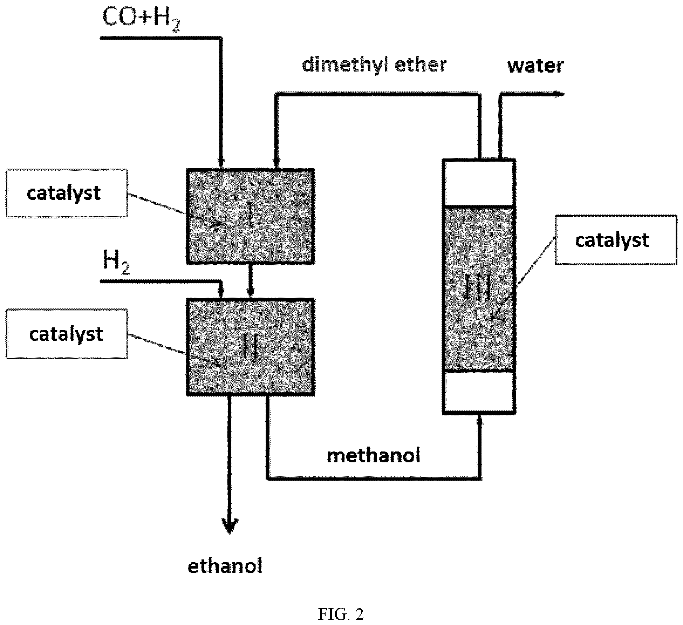

The procedure was similar to that of Example 1, except that the first reaction zone I and the first reaction zone II were located in different fixed bed reactors. Specifically, referring to FIG. 2, the reaction process was similar to the process described in Example 1 with respect to FIG. 1.

The mixed gas containing CO and H₂ together with dimethyl ether passed through the first reaction zone for reaction, and the reaction effluent entered the second reaction zone for reaction together with the addition of hydrogen. The reaction conditions were as follows: catalyst 11 # and catalyst B were filled with 3g and 7g respectively. The molar ratio of CO, DME and H₂ was 6: 1: 0.5, and the feed of dimethyl ether first entered the first reaction zone with 3g/h. The effluent from the first reaction zone and 1.43 g/h of hydrogen were added to the second reaction zone. The temperatures in the first reaction zone were 180°C, 190°C, 200°C, and 225°C, respectively. The temperature in the second reaction zone was kept at 215°C and the reaction pressure was 5 MPa. The reaction results were shown in Table 8.

**Table 8: Reaction results at different reaction temperatures in the first reaction zone**

| Reaction temperature/°C | Percent conversion of dimethyl ether (%) | Percent conversion of carbon monoxide (%) | Selectivity for methanol (%) | Selectivity for ethanol(%) | Selectivity for methyl acetate(%) | Selectivity for ethyl acetate (%) | Selectivity for others (%) |
|---|---|---|---|---|---|---|---|
| 180 | 8.5 | 1.42 | 50.1 | 49.9 | 0.0 | 0.0 | 0 |
| 190 | 25.0 | 4.17 | 50.06 | 49.88 | 0.0 | 0.0 | 0.06 |
| 200 | 35.8 | 7.50 | 55.5 | 44.3 | 0.0 | 0.0 | 0.2 |
| 225 | 75.7 | 21.95 | 63.2 | 36.2 | 0.0 | 0.0 | 0.6 |
| 280 | 98.0 | 30.3 | 64.1 | 34.8 | 0.0 | 0.0 | 1.1 |

### EXAMPLE 7

The procedure was similar to that of Example 1, except that the first reaction zone I and the first reaction zone II were located in different fixed bed reactors. Specifically, referring to FIG. 2, the reaction process was similar to the process described in Example 1 with respect to FIG. 1. The mixed gas containing CO and H₂ together with dimethyl ether passed through the first reaction zone for reaction, and the reaction effluent entered the second reaction zone for reaction together with the addition of hydrogen. The reaction conditions were as follows: catalyst 11 # and catalyst B were filled with 3g and 7g respectively. The molar ratio of CO, DME and H₂ was 6: 1: 0.5, and the feed of dimethyl ether first entered the first reaction zone with 3g/h. The effluent from the first reaction zone and 1.43 g/h of hydrogen were added to the second reaction zone. The temperature in the first reaction zone was 200°C, the temperatures in the second reaction zone were 200°C, 220°C, 240°C, and 260°C, respectively, and the reaction pressure was 5 MPa. The reaction results were shown in Table 9.

**Table 9: Reaction results at different reaction temperatures in the second reaction zone**

| Reaction temperature /°C | Percent conversion of dimethyl ether (%) | Percent conversion of carbon monoxide (%) | Selectivity for methanol (%) | Selectivity for ethanol(%) | Selectivity for methyl acetate(%) | Selectivity for ethyl acetate (%) | Selectivity for others (%) |
|---|---|---|---|---|---|---|---|
| 180 | 36.8 | 6.13 | 47.52 | 27.52 | 14.98 | 10.0 | 0.08 |
| 190 | 36.8 | 6.13 | 50.46 | 47.41 | 1.62 | 0.5 | 0.01 |
| 200 | 36.8 | 6.13 | 49.98 | 50.01 | 0.0 | 0.0 | 0.01 |
| 240 | 36.9 | 30.0 | 82.58 | 17.01 | 0.0 | 0.0 | 0.41 |
| 260 | 36.7 | 50.6 | 88.21 | 10.78 | 0.0 | 0.0 | 1.01 |
| 280 | 36.6 | 80.8 | 90.58 | 7.01 | 0.0 | 0.0 | 2.41 |

### EXAMPLE 8

The procedure was similar to that of Example 1, except that the first reaction zone I and the first reaction zone II were located in different fixed bed reactors. Specifically, referring to FIG. 2, the reaction process was similar to the process described in Example 1 with respect to FIG. 1. The reaction conditions were as follows: catalyst 11 # and catalyst B were filled with 3g and 7g respectively. The mixed gas containing CO, DME and H₂, DME entered the first reaction zone, the molar ratio of CO, DME and H₂ was 1: 1: 1, 4: 1: 1 and 10: 1: 1, respectively. The feed of dimethyl ether was 3 g/h. The effluent from the first reaction zone and 1.43 g/h of hydrogen were added to the second reaction zone. The temperature in the first reaction zone was 195°C. The temperature in the second reaction zone was kept at 215°C and the reaction pressure was 5 MPa. The reaction results were shown in Table 10.

**Table 10: Reaction results when the ratio of CO and dimethyl ether as raw materials in the first reaction zone was different**

| CO: DME | Percent conversion of dimethyl ether (%) | Percent conversion of carbon monoxide (%) | Selectivity for methanol (%) | Selectivity for ethanol (%) | Selectivity for methyl acetate (%) | Selectivity for ethyl acetate (%) | Selectivity for others (%) |
|---|---|---|---|---|---|---|---|
| 1: 1 | 6.5 | 6.62 | 50.46 | 49.54 | 0.0 | 0.0 | 0.0 |
| 4: 1 | 20.8 | 5.65 | 52.07 | 47.93 | 0.0 | 0.0 | 0.0 |
| 10: 1 | 35.6 | 4.23 | 54.30 | 45.70 | 0.0 | 0.0 | 0.0 |

The present invention has been described in detail above, but the present invention is not limited to the specific embodiments described herein. Those skilled in the art can understand that other changes and modifications can be made without departing from the scope of the invention. The scope of the invention is defined by the appended claims.

## Claims

1. A method for directly producing ethanol from syngas, whose reaction process is carried out in three reaction zones and the method comprises:
a) feeding a raw material containing syngas and dimethyl ether into a first reaction zone to contact with a solid acid catalyst in the first reaction zone, reacting to obtain an effluent containing methyl acetate and/or acetic acid;
b) allowing the effluent from the first reaction zone to enter a second reaction zone to contact with a metal catalyst in the second reaction zone and react to obtain an effluent containing methanol and ethanol;
c) separating the effluent from the second reaction zone to obtain product ethanol and by-product methanol;
d) allowing methanol from step c) to enter a third reaction zone to perform a dehydration reaction to obtain dimethyl ether, and allowing the obtained dimethyl ether to enter the first reaction zone to recycle the reaction;
wherein the volume content of syngas in the raw material is in a range from 10% to 100%, the volume content of dimethyl ether is in a range from 0% to 90%, and the volume ratio of carbon monoxide to hydrogen in the syngas is in a range from 0.1 to 10;
the reaction temperature in the first reaction zone and the second reaction zone is in a range from 180°C to 300°C, and the reaction pressure is in a range from 0.5 MPa to 20 MPa;
the reaction temperature in the third reaction zone is in a range from 180°C to 420°C, and the reaction pressure is in a range from 0.1 MPa to 4 MPa.

2. The method for directly producing ethanol from syngas of claim 1, wherein the solid acid catalyst in the first reaction zone comprises one or more molecular sieves selected from FER, MFI, MOR, ETL, MFS, MTF, EMT zeolite molecular sieves and molecular sieve products obtained by modifying the zeolite molecular sieves using pyridine or elements other than the framework constituent elements.

3. The method for directly producing ethanol from syngas of claim 2, wherein the solid acid catalyst is a hydrogen-type product of the zeolite molecular sieve, or is composed of ranging from 10% to 95% by weight of the hydrogen-type product and a remaining matrix, or is a molecular sieve product obtained by modifying the hydrogen-type product with pyridine;
wherein the matrix is one or more selected from alumina, silica, kaolin and magnesia.

4. The method for directly producing ethanol from syngas of claim 1, wherein the metal catalyst in the second reaction zone is a copper-based catalyst.

5. The method for directly producing ethanol from syngas of claim 1, wherein the first reaction zone and/or the second reaction zone are in a fixed bed reactor, and;
wherein the fixed bed reactor is preferably a tubular fixed bed reactor.

6. The method for directly producing ethanol from syngas of claim 1 or 5, wherein the first reaction zone and the second reaction zone are in the same fixed reactor, or the first reaction zone and the second reaction zone are respectively in different reactors connected in series.

7. The method for directly producing ethanol from syngas of claim 1, wherein the syngas in the raw material is composed of ranging from 50% to 100% by volume of carbon monoxide and hydrogen and ranging from 0% to 50% by volume of one or more inactive gases selected from nitrogen, helium, argon and carbon dioxide.

8. The method for directly producing ethanol from syngas of claim 1, wherein the catalyst in the third reaction zone is a solid acid catalyst for preparing dimethyl ether from methanol.

9. The method for directly producing ethanol from syngas of claim 1, wherein the third reaction zone is in a fixed bed reactor; preferably in a tubular fixed bed reactor.

10. The method for directly producing ethanol from syngas of claim 1, wherein the reaction temperature in the first reaction zone is in a range from 190°C to 290°C and the reaction pressure is in a range from 1 MP to 15 MPa; and
the reaction temperature in the second reaction zone is in a range from 190°C to 290°C and the reaction pressure is in a range from 1.0 MPa to 15.0 MPa; and
the reaction temperature in the third reaction zone is in a range from 200°C to 400°C, and the reaction pressure is in a range from 0.2 MPa to 3 MPa.
